# EUROPÄISCHE PATENTANMELDUNG

(11) **EP 0 747 076 A2**
(43) Veröffentlichungstag der Anmeldung: **11.12.1996**
(21) Anmeldenummer: 96108965.3
(22) Anmeldetag: 04.06.1996
(51) Int. Cl.: A61M 11/06

(54) **Vorrichtung zum Vernebeln von Fluiden**

(30) Priorität: 06.06.1995 DE 19520622
(71) Anmelder: MEDANZ Starnberg GmbH med. techn. Geräte, 82319 Starnberg (DE)
(72) Erfinder: Becker, Rudolf, 82319 Starnberg (DE)
(74) Vertreter: Beetz & Partner Patentanwälte

(57) **Zusammenfassung**

Die Erfindung betrifft eine Vorrichtung zum Vernebeln von Fluiden mit einem Verneblerunterteil (1)und einem zum Verneblerunterteil rotationssymmetrisch angeordneten Vernebleroberteil (2), das eine Zuluftanordnung (3) und einen Austrittsstutzen (4) aufweist. Weiterhin weist der erfindungsgemäße Vernebler einen Einsatz (5) zur Aufnahme eines Fluids (23) und eine Düsenanordnung (6) auf, die rotationssymmetrisch zur Zuluftanordnung (3) vorgesehen ist. Die Zuluftanordnung (3) weist weiterhin einen hohlzylindrischen Kanal (7) auf, welcher zur Zuluftseite hin eine konische Erweiterung (8) besitzt. Durch die erfindungsgemäße Ausbildung des Verneblers ist dieser speziell für die topische Inhalations-Anästhesie geeignet, z.B. zur Vorbereitung von bronchologischen Untersuchungen.

## Beschreibung

Die Erfindung betrifft eine Vorrichtung zum Vernebeln von Fluiden gemäß dem Oberbegriff des Patentanspruchs 1.

In der Medizintechnik sind Vorrichtungen bekannt, welche für eine Inhalationstherapie verwendet werden. Derartige Inhaliergeräte arbeiten beispielsweise Mit einer Zerstäuberdüse, die eine verjüngte Düsenbohrung aufweist. Aus dieser Düsenbohrung tritt unter Druck stehendes gasförmiges Druckmittel aus, wodurch ein flüssiges Medikament aus Ansaugkanälen angesaugt werden kann, die zu der Düsenbohrung benachbart angeordnet sind. Dieses angesaugte flüssige Medikament wird nun gegen eine gegenüber der Düsenmündung im Austrittskegel des Druckgases angeordnete Prallfläche eines Gasstromsteuers geschleudert, so daß Aerosoltröpfchen erzeugt werden. Diese Aerosoltröpfchen strömen dann gegen einen Prallschirm, welcher größere Aerosolteilchen weiter zerkleinert, so daß lungengängige (intrathorakale) Teilchengrößen von etwa 0,5 bis 5 µm erzeugt werden.

Ein derartiger Düsenvernebler ist beispielsweise aus der EP-A-0 170 715 bekannt.

Da solche Düsenvernebler jedoch Aerosoltröpfchendurchmesser von 5 µm und weniger erzeugen, sind diese nicht für Anwendungen geeignet, welche größere Tröpfchendurchmesser erfordern. Ein Beispiel einer solchen Anwendung ist etwa die topische Inhalations-Anästhesie, welche etwa bei der Durchführung bronchologischer Untersuchungen Anwendung findet. Im Gegensatz zur Inhalationstherapie werden hierbei wesentlich größere Aerosolpartikel bei einer hohen Leistung benötigt.

Gemäß dem Stand der Technik sind weiterhin Ultraschallinhalatoren bekannt, die eine Zerstäubung durch Ultraschall erzielen. Derartige Ultraschallzerstäuber weisen jedoch den Nachteil auf, daß das beschallte Medium hauptsächlich durch Kavitation einer Veränderung unterworfen wird, die bis hin zur Zerstörung des Mediums reichen kann. Durch die Kavitationseffekte können somit beispielsweise Molekülketten von Medikamenten zerstört werden, da der Ultraschall eine hohe Lokalwirkung auf das Medikament ausübt. Dadurch werden die Eigenschaften des Medikaments verändert, was wiederum bei einer Anwendung in der Anästhesiologie nicht tragbar ist.

Aufgabe der Erfindung ist es, eine Vorrichtung zum Vernebeln von Fluiden zu schaffen, die ein Tröpfchenspektrum erzeugt, welches für eine topische Inhalations-Anästhesie geeignet ist.

Diese Aufgabe wird durch die Merkmale des unabhängigen Patentanspruchs gelöst. Die abhängigen Patentansprüche geben vorteilhafte Ausführungsbeispiele und Weiterentwicklungen der Erfindung an.

Die erfindungsgemäße Vorrichtung zum Vernebeln von Fluiden weist ein Verneblerunterteil und ein damit lösbar verbundenes Vernebleroberteil auf. Die beiden Verneblerteile sind rotationssymmetrisch zueinander angeordnet. In das Verneblerunterteil kann ein Einsatz eingedrückt werden, welcher zur Aufnahme von Fluid dient. Dieses Fluid ist beispielsweise ein Anästhetikum zur Durchführung der topischen Inhalations-Anästhesie. In dem Vernebleroberteil ist eine Zuluftanordnung und ein Austrittsstutzen für erzeugte Aerosoltröpfchen vorgesehen. Die Zuluftanordnung ist rotationssymmetrisch zu dem Vernebleroberteil angeordnet und im wesentlichen durch einen hohlzylindrischen Kanal gebildet, der zur Lufteintrittsseite hin konisch erweitert ist. In dem Einsatz ist weiterhin lösbar eine Düsenanordnung vorgesehen, welche rotationssymmetrisch zu der Zuluftanordnung angeordnet ist.

Durch die erfindungsgemäße Ausbildung des Verneblers kann am Austrittsstutzen des Vernebleroberteils ein Aerosoltröpfchenspektrum erzielt werden, dessen mediale Massendurchmesserverteilung ein Maximum bei 8,5 µm aufweist. Dadurch werden gerade keine lungengängigen Aerosoltröpfchen erzeugt, sondern es ergibt sich eine Deposition der Aerosoltröpfchen im Rachen, der Glottis und den Bronchien. Somit kann beispielsweise bei der Durchführung von bronchologischen Untersuchungen eine topische Inhalations-Anästhesie mit einer hohen Effizienz durchgeführt werden. Weiterhin kann durch das Vorsehen einer Intervalltaste das Anästhetikum dosiert abgegeben werden. Dadurch können Verluste vermieden werden, da das jeweilige Präparat nur dann vernebelt wird, wenn der Patient einatmet. Weiterhin können die Teile des Verneblers aus temperaturstabilem Kunststoff gefertigt werden, so daß der Vernebler voll sterilisationsfähig ist, ausgekocht werden kann, und spülmaschinenfeste Eigenschaften aufweist.

Darüber hinaus kann der als Meßbecher verwendete Einsatz ausgetauscht werden, so daß eine Verwendung für verschiedene Vernebler möglich ist. Somit kann etwa ein Meßbecher immer mit dem gleichen Medikament befüllt werden, wodurch das Handling der Vorrichtung verbessert werden kann.

Erfindungsgemäß wird somit durch die Abstimmung der Düsenanordnung, des Einsatzes, des Vernebleroberteils mit der Zuluftanordnung und dem Austrittsstutzen aufeinander eine Anordnung geschaffen, welche ein indikationsspezifisches Tröpfchenspektrum schafft, das für die topische Inhalations-Anästhesie zur Durchführung von etwa bronchologischen Untersuchungen sehr gut geeignet ist.

Im folgenden werden Ausführungsbeispiele der Erfindung anhand von beiliegenden Zeichnungen näher erläutert. Es zeigen:
- Fig. 1: ein teilweise geschnitten dargestelltes Ausführungsbeispiel eines erfindungsgemäßen Verneblers;
- Fig. 2: eine Querschnittsansicht eines Ausführungsbeispiels eines erfindungsgemäßen Medikamentenbechers mit einer montierten Düsenanordnung;
- Fig. 3: eine Querschnittsansicht eines Ausführungsbeispiels eines erfindungsgemäßen Vernebleroberteils mit einer Zuluftanordnung und einem Austrittsstutzen und
- Fig. 4: ein teilweise geschnitten dargestelltes Ausführungsbeispiel eines erfindungsgemäßen Verneblerunterteils mit Intervalltaste.

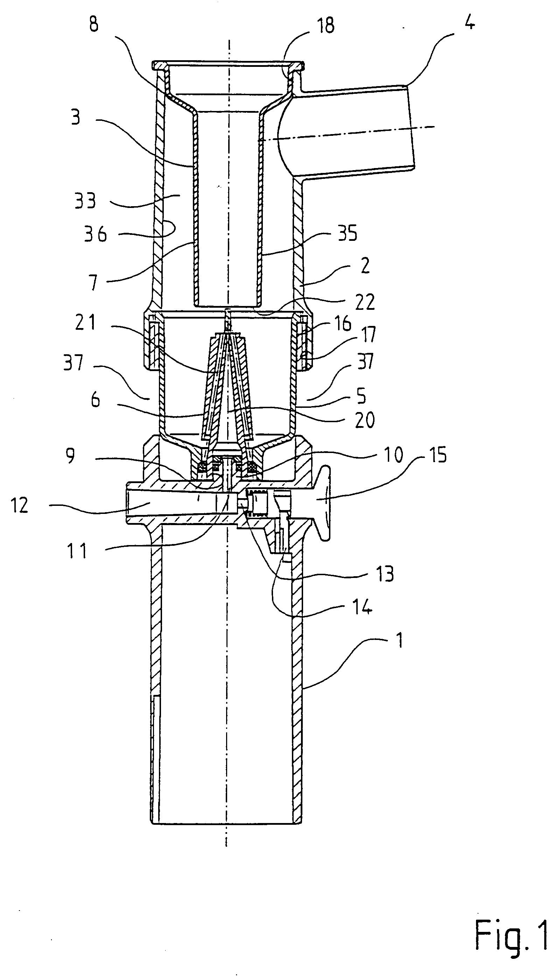

Die Fig. 1 zeigt einen Vernebler mit einem Verneblerunterteil 1 und einem Vernebleroberteil 2. In einem oberen Abschnitt des Verneblerunterteils 1 ist ein Einsatz 5 einsteckbar angeordnet. Dieser Einsatz 5 dient unter anderem als Medikamentenbecher. In dem Einsatz 5 ist eine Düsenanordnung 6 vorgesehen. Die Düsenanordnung 6 weist eine Bohrung 9 auf, welche mit einem Zapfen 10 in Eingriff steht. Der Zapfen 10 weist wiederum eine Innenbohrung 11 auf, die mit einem Zuleitungskanal 12 für Druckluft in Verbindung steht. Der Zuleitungskanal 12 ist weiterhin über eine Verbindungsbohrung 13 mit einer Luftaustrittsöffnung 14 verbunden. Durch eine im Verneblerunterteil 1 vorgesehene Intervalltaste 15 kann die Luftaustrittsöffnung 14 verschlossen werden, so daß die über den Zuleitungskanal 12 zugeführte Druckluft über die Innenbohrung 11 des Zapfens 10 in einen Druckluftkanal 20 der Düsenanordnung 6 strömt.

Der obere Abschnitt des Verneblerunterteils weist weiterhin beispielsweise zwei Öffnungen 37 auf, durch die der Blick auf den Einsatz 5 bzw. den Medikamentenbecher freigegeben wird. Da der Medikamentenbecher vorzugsweise aus einem transparenten Material gefertigt ist, kann dadurch der jeweilige Füllstand des Medikamentenbechers überprüft werden.

In das Vernebleroberteil 2 ist eine Zuluftanordnung 3 eingesetzt, die einen hohlzylindrischen Kanal 7 an der Luftaustrittsseite der Zuluftanordnung 3 und eine konische Erweiterung 8 an der Lufteintrittsseite der Zuluftanordnung 3 aufweist. An dem Vernebleroberteil 2 ist weiterhin ein hohlzylindrisch ausgeformter Austrittsstutzen 4 angeformt, welcher mit einem nicht dargestellten Mundstück in Verbindung gebracht werden kann. Durch dieses Mundstück kann der Patient die durch die Düsenanordnung 6 erzeugten Aerosoltröpfchen inhalieren.

Der erfindungsgemäße Vernebler kann durch den vorteilhaften Aufbau seiner Teile einfach zusammengesteckt bzw. zusammengesetzt werden. Beginnend etwa bei dem Verneblerunterteil 1 mit der schon vorgesehenen Intervalltaste 15 kann der Einsatz 5 mit eingesteckter Düsenanordnung 6 auf den Zapfen 10 aufgesetzt werden, bzw. in den oberen Abschnitt des Verneblerunterteils 1 eingesetzt werden. Der Einsatz 5 kann etwa schon vor dem Einsetzen in das Verneblerunterteil 1 mit dem entsprechenden Anästhetikum befüllt werden, es ist jedoch auch möglich das Anästhetikum erst bei einem eingesetzten Einsatz 5 in denselben zu dosieren.

Anschließend kann das Vernebleroberteil 2 auf dem Verneblerunterteil 1 befestigt werden. Die Verbindung kann über eine Verschraubung erfolgen, wobei ein an dem Verneblerunterteil 1 angeordnetes Außengewinde 16 in ein am Vernebleroberteil 2 angeordnetes Innengewinde 17 eingreift. Das Vernebleroberteil 2 weist die Zuluftanordnung 3 auf, welche lösbar in das Vernebleroberteil 2 eingesteckt ist. Die eingesteckte Zuluftanordnung 3 steht im zusammengebauten Zustand in einer Formschlußverbindung eines Abschnitts der Erweiterung 8 mit einem Innenumfangsabschnitt 18 des Vernebleroberteils 2. In einem oberen Bereich des Vernebleroberteils 2 ist der Austrittsstutzen 4 angeordnet, welcher etwa mit einem Winkel von vorzugsweise 5° von einer Normalen zur Symmetrieachse des Vernebleroberteils 2 nach oben geneigt ist. Über diesen Austrittsstutzen 4 kann dann das nicht dargestellte Mundstück geschoben werden.

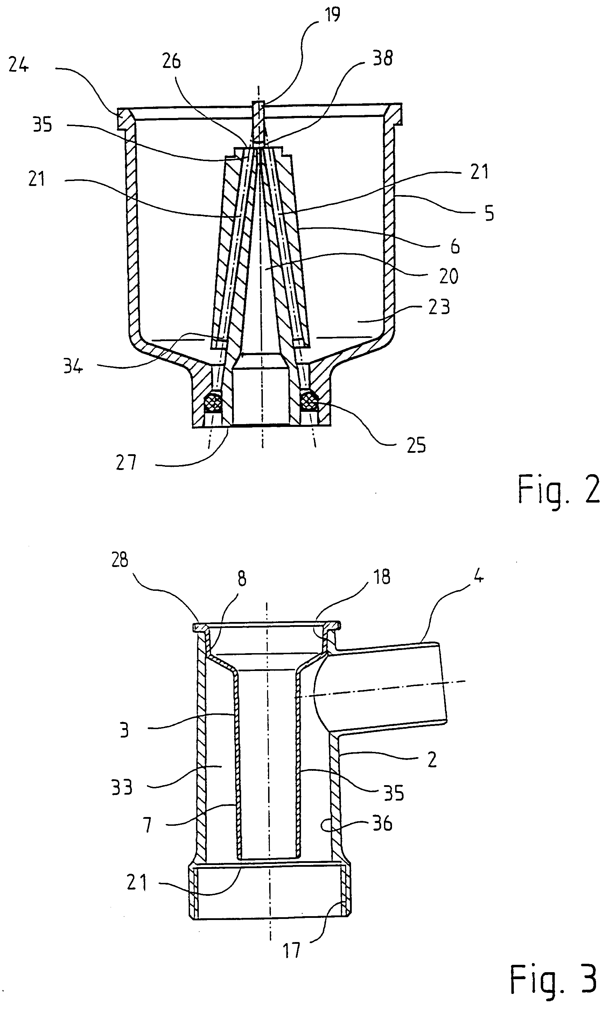

Im Betrieb des Verneblers wird dem Zuleitungskanal 12 Druckluft zugeführt. Wenn die Intervalltaste 15 nicht betätigt wird, strömt diese zugeführte Druckluft aus der Luftaustrittsöffnung 14 aus. Wird nun die Intervalltaste 15 betätigt, strömt die zugeführte Druckluft durch den Druckluftkanal 20 der Düsenanordnung 6 und tritt durch eine Düse 38 der Düsenanordnung 6 aus (siehe Fig. 2). Die aus der Düse 38 ausströmende Druckluft wird von einem Gasstromsteuer 19 derart abgelenkt, daß in Ansaugkanälen 21 der Düsenanordnung 6 ein Unterdruck entsteht. Ein unteres Ende 34 des Ansaugkanals 21 öffnet in einen unteren Bereich des Einsatzes 5. Im Betrieb ist der Einsatz 5 mit Fluid derart gefüllt, daß das untere Ende 34 des Ansaugkanals 21 mit Fluid bedeckt ist. Durch den an einem oberen Ende 35 des Ansaugkanals 21 entstehenden Unterdruck wird nun Fluid aus dem Einsatz 5 in die Ansaugkanäle 21 gesaugt und durch die durch die Düse 38 austretende Druckluft fein verteilt. Die dabei entstehenden Aerosolpartikel weisen vorzugsweise eine mediane Massendurchmesserverteilung auf, die ihr Maximum bei 40 µm hat. Diese erzeugten Aerosolpartikel werden nun beispielsweise durch ein Aufprallen auf einen unteren Rand 22 des hohlzylindrischen Kanals 7 und durch Aufprallvorgänge auf die Außenwand des hohlzylindrischen Kanals 7 und die Innenwand des Vernebleroberteils 2 weiter zerkleinert.

Für eine Inhalation der erzeugten Aerosolpartikel erzeugt der Patient über das Mundstück einen Unterdruck an dem Austrittsstutzen 4, wodurch eine Luftströmung durch die Zuluftanordnung 3 von oben nach unten in Richtung auf die Düsenanordnung 6 entsteht. Diese Luft strömt dann durch einen Ringkanal 33 von unten nach oben zu dem Austrittsstutzen 4. Der Ringkanal 33 wird im wesentlichen von einer Außenfläche 35 der Zuluftanordnung 3 und einer Innenfläche 36 des Vernebleroberteils 2 gebildet.

Durch die geometrische Ausbildung und Abstimmung der einzelnen Bauteile aufeinander, insbesondere der Düsenanordnung 6, der Zuluftanordnung 3, der Innenwandung des Vernebleroberteils 2 und der Außenwandung des Austrittsstutzens 4 können Aerosoltröpfchen mit einer medianen Massendurchmesserverteilung erzeugt werden, die ein Maximum vorzugsweise bei 8,5 µm aufweist. Dadurch ist der erfindungsgemäße Vernebler ganz besonders für eine topische Inhalations-Anästhesie etwa zur Durchführung von bronchologischen Untersuchungen geeignet.

Bei der gewählten Geometrie hat es sich als vorteilhaft herausgestellt, daß das Druckgas vorzugsweise einen Überdruck von 1,1 · 10⁵ Pa aufweist, wodurch ein Gesamt-Output von etwa 800 mg/min erzeugt werden kann.

In der Fig. 2 ist eine vergrößerte Darstellung des Einsatzes 5 mit der Düsenanordnung 6 gezeigt. Der Einsatz 5 ist mit einem Fluid 23 teilweise befüllt. Bei diesem Fluid 23 handelt es sich beispielsweise um das zum Einsatz kommende Anästhetikum. In dieser Figur ist dargestellt, daß im Betrieb die Oberkante des Fluids 23 über der unteren Öffnungen 34 der Ansaugkanäle 21 angeordnet ist. Dadurch kann das Fluid 23 in die Ansaugkanäle 21 eingesaugt werden, um dann aus entsprechenden oberen Öffnungen 26 auszutreten. Anschließend wird das Fluid 23 durch den aus der Düse 38 und von der Gasstromsteuer 19 abgelenkten Gasstrom zerstäubt. Der Medikamentenbecher kommt im in das Vernebleroberteil 2 eingesetzten Zustand über eine Abstufung 24 auf einem oberen Rand des Verneblerunterteils zu liegen. Weiterhin ist der Einsatz 5 durch eine untere Auflagefläche 27 mit dem Verneblerunterteil 1 in Kontakt. Die Düsenanordnung 6 ist rotationssymmetrisch zum Einsatz 5 angeordnet. Zwischen der Düsenanordnung 6 und dem Einsatz 5 ist ein O-Ring 25 angeordnet. Der O-Ring 25 dichtet die Düsenanordnung 6 gegen den Einsatz 5 ab.

In der Fig. 3 ist das Vernebleroberteil 2 dargestellt. Das Vernebleroberteil 2 ist im wesentlichen zylindrisch ausgebildet und weist an seiner unteren Seite das Innengewinde 17 auf. An dem Innenumfangsabschnitt 18 des Vernebleroberteils 2 ist formschlüssig die Zuluftanordnung 3 mit der konischen Erweiterung 8 angeordnet. Die Zuluftanordnung 3 weist am oberen Ende einen Überstand 28 auf, der sich gegen den oberen Rand des Vernebleroberteils 2 abstützt. Die Zuluftanordnung 3 kann somit in das Vernebleroberteil 2 eingesteckt werden, wobei es durch den Überstand 28 zu einer definierten Lage kommt. Im eingesteckten Zustand ist die Zuluftanordnung 3 rotationssymmetrisch zu dem Vernebleroberteil 2 angeordnet. An dem Vernebleroberteil 2 ist der Stutzen 4 angeformt. Durch den hohlzylindrischen Kanal 7 der Zuluftanordnung 3 wird der Ringkanal 33 zwischen der Außenfläche 35 des hohlzylindrischen Kanals 7 und der Innenfläche 36 des Vernebleroberteils 2 gebildet. Die in diesem ringförmigen Strömungsraum entstehenden fluidmechanischen Vorgänge tragen unter anderem dazu bei, daß der bevorzugte Aerosoltröpfchendurchmesser erzeugt wird. Die Dimensionierung dieser Bauteile ist etwa derart gewählt, daß ein auf einer mittleren Höhe abgenommenes Durchmesserverhältnis zwischen dem Innendurchmesser des Vernebleroberteils 2 und dem Außendurchmesser des hohlzylindrischen Kanals 7 vorzugsweise etwa 1,8 betragen kann. Die Wandstärke des hohlzylindrischen Kanals 7 kann dabei etwa 1 mm betragen und deren Innendurchmesser etwa auf halber Höhe 1,45 mm. Der hohlzylindrische Kanal 7 kann etwa eine Länge von 4,6 mm aufweisen und sich zur Luftaustrittsseite hin leicht konisch verjüngen.

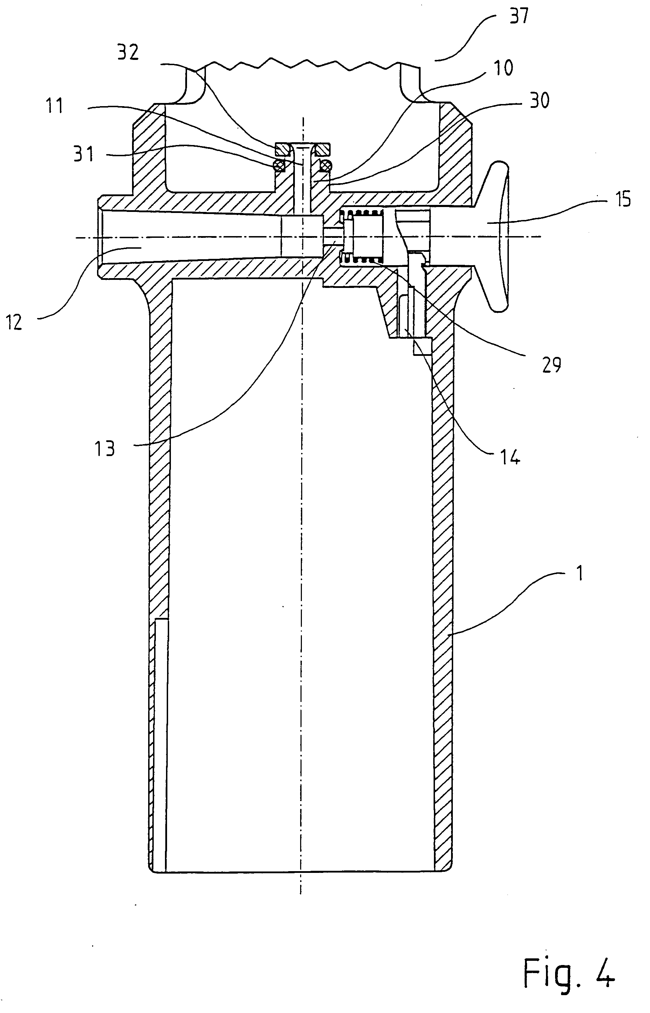

Die Fig. 4 zeigt das Verneblerunterteil 1, welches in seinem unteren Abschnitt im wesentlichen hohlzylindrisch ausgebildet ist. In einem oberen Abschnitt des Verneblerunterteils 1 weist dasselbe die Intervalltaste 15 auf. Gegenüberliegend zur Intervalltaste 15 ist der Zuleitungskanal 12 für die Druckluft vorgesehen. Dieser Zuleitungskanal 12 steht zum einen mit der Innenbohrung 11 des Zapfens 10 in Verbindung und zum anderen mit einer Verbindungsbohrung 13 zur Luftaustrittsöffnung 14. Im nichtbetätigten Zustand der Intervalltaste 15 wird dieselbe durch eine Druckfeder 29 bis zu einem Anschlag nach außen gedrückt, so daß eine Fluidverbindung zwischen dem Zuleitungskanal 12, der Verbindungsbohrung 13 und der Luftaustrittsöffnung 14 erzeugt wird. Im gedrückten Zustand der Intervalltaste 15 wird die Luftaustrittsöffnung 14 bzw. Die Verbindungsbohrung 13 verschlossen, so daß das in den Zuleitungskanal 12 strömende Druckmedium durch die Innenbohrung 11 in den Zapfen 10 und damit in die Düsenanordnung 6 strömt. Der Zapfen 10 weist eine Stufe 30 auf, an der ein O-Ring 31 angeordnet ist. Wenn die in Fig. 2 dargestellte Anordnung auf den Zapfen 10 aufgesteckt wird, vgl. auch Fig. 1, dichtet der O-Ring 31 die Düsenanordnung 21 gegen den Zapfen 10 ab. Weiterhin ist an dem Zapfen 10 oben eine ringförmige Rastscheibe 32 ausgeformt. Die Rastscheibe 32 dient zum einen für eine Fixierung des O-Rings 31 derart, daß dieser nicht bei einem Abziehen der Düsenanordnung 6 von dem Zapfen rutscht und zum anderen für eine Verrastung der Düsenanordnung 6 im eingesetzten Zustand.

Der erfindungsgemäße Vernebler ist insbesondere für eine Verwendung bei einer topischen Inhalations-Anästhesie geeignet, da Aerosoltröpfchendurchmesser erzeugt werden, die zu einer Deposition im Rachen-, Glottis- und Bronchienraum führen. Weiterhin wird ein Tröpfchenspektrum erzeugt, welches nicht lungengängig ist, da ein solches Tröpfchenspektrum für eine Inhalations-Anästhesie ungünstig ist. Durch das indikationsspezifische Tröpfchenspektrum eignet sich die erfindungsgemäße Vorrichtung etwa bevorzugt für die Vorbereitung von bronchologischen Untersuchungen, wobei der beschriebene Vernebler für eine eingriffsvorbereitende topische Inhalations-Anästhesie bevorzugt zur Anwendung kommen kann.

## Patentansprüche

1. Vorrichtung zum Vernebeln von Fluiden mit
- einem Verneblerunterteil (1),
- einem zum Verneblerunterteil (1) rotationssymmetrisch angeordneten Vernebleroberteil (2), das eine Zuluftanordnung (3) zum Zuführen von Zusatzluft und einen Austrittsstutzen (4) zum Abführen von vernebeltem Fluid (23) aufweist,
- einem Einsatz (5) zur Aufnahme des Fluids (23) und
- einer Düsenanordnung (6), die rotationssymmetrisch zur Zuluftanordnung (3) vorgesehen ist,
**dadurch gekennzeichnet, daß**
die Zuluftanordnung (3) zur Luftaustrittsseite hin einen hohlzylindrischen Kanal (7) aufweist.

2. Vorrichtung nach Anspruch 1,
dadurch gekennzeichnet, daß
die Zuluftanordnung (3) zur Lufteintrittsseite hin eine konische Erweiterung (8) aufweist.

3. Vorrichtung nach Anspruch 1 oder 2,
dadurch gekennzeichnet, daß
das Verneblerunterteil (1) mit dem Vernebleroberteil (2) lösbar verbunden ist.

4. Vorrichtung nach zumindest einem der Ansprüche 1 bis 3,
dadurch gekennzeichnet, daß
das Verneblerunterteil (1) den Einsatz (5) lösbar aufnimmt und die Düsenanordnung (6) lösbar in dem Einsatz (5) angeordnet ist.

5. Vorrichtung nach zumindest einem der Ansprüche 1 bis 4,
dadurch gekennzeichnet, daß
das Verneblerunterteil (1) einen Zapfen (10) aufweist, der in eine Bohrung (9) der Düsenanordnung (6) eingreift, wenn der Einsatz (5) mit der Düsenanordnung (6) in das Verneblerunterteil (1) eingesetzt ist.

6. Vorrichtung nach zumindest einem der Ansprüche 1 bis 5,
dadurch gekennzeichnet, daß
der Zapfen (10) eine Innenbohrung (11) aufweist, die mit einem Zuleitungskanal (12) für Druckluft in Verbindung steht, wobei bei einem in das Verneblerunterteil (1) eingesetzten Einsatz (5) mit der eingesetzten Düsenanordnung (6) eine Fluidverbindung zwischen dem Zuleitungskanal (12) und einem Druckluftkanal (20) der Düsenanordnung (6) entsteht und daß eine Intervalltaste (15) zur Unterbrechung des Druckluftstroms vorgesehen ist.

7. Vorrichtung nach zumindest einem der Ansprüche 1 bis 6,
dadurch gekennzeichnet, daß
der Einsatz (5) aus einem teilweise transparenten Material gebildet ist und das Verneblerunterteil (1) zumindest eine Öffnung (37) aufweist, die den Blick auf den Einsatz (5) freigibt.

8. Vorrichtung nach zumindest einem der Ansprüche 1 bis 7,
dadurch gekennzeichnet, daß
die Düsenanordnung (6) zwei Ansaugkanäle (21) für das Fluid (23) und ein Gasstromsteuer (19) aufweist, wobei durch die Düsenanordnung (6) Aerosolteilchen mit einer medianen Massendurchmesserverteilung erzeugt werden, die ein Maximum vorzugsweise bei 40 µm aufweist.

9. Vorrichtung nach zumindest einem der Ansprüche 1 bis 8,
dadurch gekennzeichnet, daß
die Düsenanordnung (6), die Zuluftanordnung (3) des Vernebleroberteils (2), die innere Oberfläche des Vernebleroberteils (2) und ein zwischen einer Außenfläche (35) der Zuluftanordnung (3) und einer Innenfläche (36) des Vernebleroberteils (2) gebildeter Ringkanal (33) derart ausgebildet und aufeinander abgestimmt sind, daß Aerosoltröpfchen mit einer medianen Massendurchmesserverteilung erzeugt werden, die ein Maximum vorzugsweise bei 8,5 µm aufweist.

10. Vorrichtung nach zumindest einem der Ansprüche 1 bis 9,
dadurch gekennzeichnet, daß
das Druckgas vorzugsweise einen Überdruck von 1,1 · 10⁵ Pa aufweist und ein Gesamt-Output von vorzugsweise 800 mg/min erzeugt wird.

11. Verwendung der Vorrichtung nach zumindest einem der Ansprüche 1 bis 10 für eine topische Inhalations-Anästhesie, insbesondere zur Durchführung von bronchologischen Untersuchungen.
